# EUROPEAN PATENT APPLICATION

(11) **EP 1 914 320 A2**
(43) Date of publication of application: **23.04.2008**
(21) Application number: 07116974.2
(22) Date of filing: 21.09.2007
(51) Int. Cl.: C12Q 1/68

(54) **A method for detection of one or more CpG positions**

(30) Priority: 16.10.2006 EP 06122337
(71) Applicant: Epigenomics AG, 10178 Berlin (DE)
(72) Inventor: Schuster, Matthias, Dr., 13156 Berlin (DE); Schatz, Philipp, Dr., 10435 Berlin (DE); Heiden, Esmeralda, Dr., 10589 Berlin (DE)

(57) **Abstract**

The invention relates generally to novel and substantially improved methods for detecting CpG positions. Particular aspects relate to a method for detection of one or more CpG positions. Said method comprises: providing a sample, binding a protein or peptide onto the DNA of said sample, hybridizing a probe onto the DNA of said sample, detecting a signal determined by said bound proteins or peptides and by said hybridized probes. Thereby one or more CpG positions are detected in a methylation specific and sequence specific manner.

Sequence listing

<110> Epigenomics AG

<120> A METHOD FOR DETECTION OF ONE OR MORE CpG POSITIONS

<160> 3

<210> 1
<211> 40
<212> DNA
<213> Homo Sapiens

<400> 1

gtgttgactt tgagttgttt tcctgctgtc tgtttactcc                    40

<210> 2
<211> 60
<212> DNA
<213> Homo Sapiens

<400> 2

aagtcaacac tttttttttt atgttaagtg accggcagca tttttttttt aaacaactca      60

<210> 3
<211> 20
<212> DNA
<213> Homo Sapiens

<400> 3

tgctgccggt cacttaacat                    20

## Description

### FIELD OF THE INVENTION

The invention relates generally to novel and substantially improved methods for detecting CpG positions. In particular it relates to a methylation specific detection of at least one CpG positions, wherein each position is associated with a specific genomic sequence.

### BACKGROUND OF ASPECTS OF THE INVENTION

*DNA methylation:* Many diseases, in particular cancer diseases, are accompanied by modified gene expression. This may be related to a mutation of the genes themselves, which leads to an expression of modified proteins or to an inhibition or over-expression of the proteins or enzymes. A modulation of gene expression may, however, also occur by epigenetic modifications, and in particular by DNA methylation. Such epigenetic modifications do not alter the actual DNA coding sequence, but nonetheless have substantial health implications, and it is clear that knowledge about methylation processes and modifications of methylation related metabolism and DNA methylation are essential for understanding, prophylaxis, diagnosis and therapy of diseases.

Cytosine methylation of CpG dinucleotides by S-adenosylmethionine (SAM)-dependent DNA methyltransferases represent one mechanism for gene regulation. Genes can be transcribed by methylation-free promoters, even when adjacent transcribed or non-transcribed regions are widely methylated. This permits the use and regulation of promoters of functional genes, whereas non-gene associated DNA including the transposable elements is suppressed. Methylation is also involved in the long-term suppression of X-linked genes, and may lead to either a reduction or an increase of the degree of transcription, depending on where the methylation in the transcription unit occurs.

CpG dinucleotides represent about 1 to 2% of all dinucleotides and are concentrated in so-called CpG islands. A CpG island is usually defined in the art as a DNA region of about 200 bp having a CpG content of at least 50%, and where the ratio of the number of observed CG dinucleotides and the number of the expected CG dinucleotides is larger than 0.6 (Gardiner-Garden, M., Frommer, M. (1987) J. Mol. Biol. 196, 261-282). Typically, CpG islands have at least 4 CG dinucleotides in a sequence having a length of 100 base pairs. It is known in the art that CpG positions can be co-methylated or co-unmethylated. This is in particular the case for CpG positions of CpG islands (cf. EP 06090110; Rakyan VK, Hildmann T, Novik KL, Lewin J, Tost J, Cox AV, Andrews TD, Howe KL, Otto T, Olek A, Fischer J, Gut IG, Berlin K, Beck S. DNA methylation profiling of the human major histocompatibility complex: a pilot study for the human epigenome project PLoS Biol. 2004, 2(12):e405; Eads CA, Danenberg KD, Kawakami K, Saltz LB, Blake C, Shibata D, Danenberg PV, Laird PW. MethyLight: a high-throughput assay to measure DNA methylation. Nucleic Acids Res. 2000 Apr 15;28(8):E32).

Hypermethylation often leads to the suppression of the expression. In the normal state, a tumor suppressor gene is hypomethylated. If a hypermethylation takes place, this will lead to a suppression of the expression of the tumor suppressor gene, which is frequently observed in cancer tissues. In contrast thereto, oncogenes are hypermethylated in healthy tissue, whereas in cancer tissue they are frequently hypomethylated.

Cytosine methylation typically prevents the binding of proteins regulating transcription. This leads to a modification of associated gene expression. In the context of cancer, for example, the expression of cell division regulating genes is thereby affected (e.g., the expression of apoptosis genes is down-regulated, whereas oncogene expression is up-regulated). DNA hypermethylation also has a long-term influence on gene regulation. Via cytosine methylation, histone de-acetylation proteins can bind to the DNA by their 5-methyl cytosine-specific domain. Consequently, histones are de-acetylated, leading to a tighter DNA compaction, whereby regulatory proteins are precluded from DNA binding.

Because of the said above, the detection of CpG methylation or CpG non-methylation is important with respect to diagnosing a disease, prognosing a disease, predicting a treatment response, diagnosing a predisposition for a disease, diagnosing a progression of a disease, grading a disease, staging a disease, classifying a disease, characterizing a disease, or for identifying a new marker associated with a disease. Additionally, the effects of a therapy can be monitored in case of a diseased individual. Most of the known methods for methylation analysis are only applicable to isolated genomic DNA. An overview of said methods can be gathered from Laird PW. "The power and the promise of DNA methylation markers" Nat Rev Cancer 2003 Apr;3(4):253-66. Many methods for methylation analysis are based on treatment of genomic DNA with reagent that differentiates between methylated and unmethylated cytosines. In many cases this reagent is a bisulfite reagent which leads to a conversion of unmethylated cytosines to uracil or after amplification to thymin while methylated cytosines remain unchanged. Other methods are based on the selected digestion by means of restriction enzymes either methylation sensitive or non-methylation sensitive. Exemplary, one of such methods is described in PCT/EP2006/064408. Said method is a method for sensitive detection of methylated or unmethylated CpG dinucleotides only out of body fluid samples and not out of tissue samples. Accordingly, (i) a biological sample is removed; (ii) DNA of the sample is enriched; (iii) the enriched DNA is methylation specifically converted by means of chemical or enzymatic treatment; (iv) the converted DNA is amplified; and (v) the amplified DNA is analyzed. The said steps of the method are carried out in the order (i), (ii), (iii), (iv), (v) or in the order (i), (iii), (ii), (iv), (v). In any case, DNA is isolated, enriched and predominantly converted, before it is methylation and sequence specifically analyzed.

However, in general, a pronounced need in the art exists for methods for methylation analysis of genomic DNA, wherein the morphology of a sample is maintained.

*Prior art:* Pathological, histochemical, or cell biological methods in general allow a spatially resolved analysis of distinct cells. This is particularly import, if distinct cells have to be analyzed, only a small number of cells is analyzed in the presence of numerous other cells, or if the distribution of cells or their distribution pattern is of interest. The advantages of pathological, histochemical or cell biological methods as well as their utility are well known in the art and are therefore not described in further detail.

Currently the applicant is only aware of two method which allows a pathological, histochemical or cell biological analysis of DNA methylation. The first method is known as the in situ MSP method (Nuovo GJ., Methylation-specific PCR in situ hybridization. Methods Mol Biol. 2004;287:261-72.). According to Nuovo, either de-waxed paraffin-embedded, formalin-fixed tissue or a formalin-fixed cell preparation is used. After treatment with a protease, genomic DNA is bisulfite treated. Subsequent to in situ PCR amplification, the amplicons are detected by hybridization to probes labeled with biotin, binding of streptavidin-alkaline phosphatase and by colorimetric reaction of NBT/BCIP.

However, the in situ MSP method has several very limiting disadvantages. First of all, the said method requires a lot of personal handling skills and experience of the experimenter. This is also emphasized by Nuovo (Nuovo GJ, supra). Secondly, the amplificates of the MSP reaction are not localized at the site of amplification. Instead they are mobile and can diffuse away, leading to false positives results. Thirdly, the MSP reaction provides only qualitative results. The quantification of the DNA-methylation is not possible. Fourthly, the results of the in situ MSP method are highly dependent on several very critical factors. These factors are dependent on each other and on the tissue or cells which have to be analyzed. A subgroup of factors relates to fixation of the tissue or cells. The in situ MSP method is limited to a fixation of tissue or cells with formalin. Other possibilities of fixation lead to an inhibition of subsequent steps or false results. Such fixations are for example fixation by means of picric acid or of heavy metal ions. Also critical is the extent of fixation i.e. the concentration of applied fixative and the duration of fixation. A strong fixation is necessary to enable the obtainment of staining results with a still analyzable morphology. On the other side, a strong fixation hinders or inhibits the necessary diffusion of chemical reagents, primers, polymerase or probes of subsequent methods steps. This has the effect, that the respective reactions do not take place and false results are obtained. In addition, fixation also has the effect of cross linking the double stranded genomic DNA. Because of this, single stranded genomic DNA can only insufficiently be generated. But, genomic DNA has to be present in single stranded form to be converted by bisulfite. Remaining double stranded DNA is not converted by bisulfite. Therefore, unconverted cytosines of this regions will mistakenly be detected and considered as methylated although they are non-methylated (false positive results). A further subgroup of factors relates to making the genomic DNA accessible for bisulfite treatment, MSP and hybridization. Therefore the sample is digested with a protease. This loosens the scaffold generated by fixation. But it has also easily the effect of obtaining staining results of poorly analyzable morphology. The third subgroup of factors relate to bisulfite treatment. In particular, the treatment with bisulfite is a very harsh treatment, destroying much of the tissue's or cell's morphology. This makes a strong fixation necessary. But such a fixation has on the other hand a negative effect on the melting of double stranded DNA (see above). Therefore genomic DNA is made insufficiently accessible for bisulfite treatment, which itself leads to false positive results. Because the said factors are also strongly dependent on the texture of the analyzed tissue or group of cells, a lot of optimization is also necessary. In addition, it was not possible for the applicant or the inventors to reproduce the method as described in Nuovo GJ (supra).

The second method for the spatial resolved analysis of methylation is described in Stains et al. (Stains Cl, Furman JL, Segal DJ, and Ghosh I. Site-specific detection of DNA methylation utilizing mCpG-SEER. J Am Chem Soc. 2006 Aug 2;128(30):9761-5). According to it, two fusion proteins are applied onto a sample. The first fusion protein comprises a zinc-finger domain and one half of a GFP. This protein is targeted to specific sequences. The other fusion protein comprises a methyl-CpG binding domain protein and the complementary half of said GFP. Wherein a methylated cytosine appears in a defined distance to said specific sequence, a signal is derived generated by the two halfs of the GFP. However this method has several disadvantages. In particular, it is limited to sequences to which zinc-finger domain can bind. In addition, it also not very sensitive because only two re-assembled GFP molecules per cell give rise to a signal.

Because of the disadvantages of the prior art methods and because of the large benefits which would be obtainable by an easy to use, and reliable method requiring not excessive optimization, a greet need in the art exists for such new methods for site directed methylation-specific detection of CpG positions.

### DETAILED DESCRIPTION OF ASPECTS OF THE INVENTION

For achieving various technical objects, particular aspects of the invention teach and provide a method for detection of one or more CpG positions, comprising:
(a) providing a sample, said sample has a conserved morphology and comprises genomic DNA;
(b) binding at least one protein or peptide onto the provided genomic DNA, wherein said binding is dependent on the methylation status of the binding site;
(c) hybridizing at least one probe onto the provided genomic DNA, wherein said hybridizing is dependent on the sequence of the hybridization site; and
(d) detecting at least one signal, each of said signals being determined by one or more of said bound proteins or peptides and by one or more of said hybridized probes, wherein said one or more CpG positions are detected in a methylation specific and sequence specific manner.

Particular aspects of the invention teach and provide a method for detection of one or more CpG positions. Said method being characterized in that genomic DNA is provided, a protein or peptide is methylation specifically bound to the DNA, a probe hybridizes locus specifically to said genomic DNA, and a signal is detected being determined by the binding of said protein or peptide and the hybridizing of said probe. Particular aspects of the invention teach and provide a method for detection of one or more CpG positions, wherein said one or more CpG positions are part of a repetitive sequence element. Particular aspects of the invention teach and provide a method for detection of one or more CpG positions wherein a signal is enhanced by means of secondary antibodies, ligands and ligand binding proteins, and/or amplification like PCR or isothermal amplification. Particular aspects of the invention teach also a kit for detection of one or more CpG positions. Further particular aspects of the invention teach the use of the herein taught and provided methods as well as the herein taught kit.

### Advantages of aspects of the invention

The herein provided method of the invention is characterized in that it enables the spatially resolved methylation analysis of tissues or cells. It is easy to be performed because no particular handling skills nor difficult method steps are applied. The method comprises amongst others well known standard techniques put into a new order. Therefore, also no pre-experience is necessary. Because of its simplicity, it is highly reliable and reproducible.

Particular aspects of the invention are further characterized in comprising the use of fixed starting material or the fixation of tissue or cells. The thereby used fixative can be any fixative and it can be applied in wide variety of conditions. Said fixative or conditions are only limited in that the 5'-methylcytosine is still recognizible by the 5'-methylcytosine binding protein or peptide and in that the probe is still able to hybridize sequence specifically to the DNA after fixation. Because the said two limitations are quite weak, a lot of different fixatives and fixation methods are possible. This is in clear contrast to the prior art which allows only a fixation by formalin.

Particular aspects of the invention are further characterized in comprising a digestion by a protease. Such a digestion is quite uncritical, because only a mild digestion is necessary. The morphology will be conserved. Alternatively and even milder, the digestion step is replaced by a heating step.

Particular aspects of the invention are also characterized in that the signal which is generated and detected is localized and not free-floating as it is the case according to the prior art. This localized signal enables a better spacial resolution and prevents false results.

The method of the invention has further the advantage that it is independent from the cell type or tissue. This is based therein that only minor requirements are made on the permeability of the sample. Different kinds of cell types or tissues of a wide variety of thicknesses can be used according to the invention. Correspondingly, only the fixation duration will be extended or shortened according to standard procedures known to those skilled in the art.

Because fixation as well as protease digestion or heating are so uncritical factors, permeability of the sample and morphology preservation are not an issue. Therefore, the conditions of the method of the invention are selectable in a way they enable histochemical staining with an optimal preserved morphology.

The method of the invention has further the advantage that it does not require a treatment with bisulfite or any other DNA converting chemical reagent as it is required according to prior art methods. Because such treatments are very harsh, the obtained stainings have a usually bad morphology. Because this is not necessary according to the invention, the resulting stainings have an improved morphology in comparison to stainings obtained by prior art methods. In addition, the absence of a bisulfite treatment has also the advantage that it is not possible to receive any false positive results because of an incomplete bisulfite conversion. An incomplete bisulfite conversion means that only parts of genomic DNA are converted and other remain unconverted. Thus unmethylated cytosines of a CpG dinucleotide will be detected as methylated although they are unmethylated.

In addition, the method of the invention has the advantage that embodiments can be easily transferred from one tissue or cell type to another tissue or cell type. Furthermore, also said embodiments can also be easily transferred from one genomic position to another genomic position. Therefore, it has to be chosen only a corresponding sequence specific probe with similar chemical or physical properties.

### Method of aspects of the invention

Aspects of the present invention relate to a method for detecting at least one CpG position. Thereby the detection depends on the methylation status of the corresponding cytosines i.e. the cytosine(s) of the CpG position(s) being methylated or unmethylated. The cytosines of said CpG positions can either be co-methylated or not. The signal derived by the method of the invention is enhanced, wherein the cytosines are co-methylated. In addition, said detection depends also on the sequence or sequences of which the one or more CpG positions are part of.

The method of the invention is a method for detection of one or more CpG positions, comprising:
(a) providing a sample, said sample has a conserved morphology and comprises genomic DNA;
(b) binding at least one protein or peptide onto the provided genomic DNA, wherein said binding is dependent on the methylation status of the binding site;
(c) hybridizing at least one probe onto the provided genomic DNA, wherein said hybridizing is dependent on the sequence of the hybridization site;
(d) detecting at least one signal, each of said signals being determined by one or more of said bound proteins or peptides and by one or more of said hybridized probes, wherein said one or more CpG positions are detected in a methylation specific and sequence specific manner.

According to aspects of the invention, a cytosine of a CpG position or two, at least two, three, four, five, seven, ten, fifteen, twenty, fifty, one hundred, one thousand or more cytosines of different CpG positions are detected methylation specifically and sequence specifically. It is also feasible to perform a genome wide DNA methylation analysis. Therefore at least the following steps are carried out: (i) providing a sample, said sample has a conserved morphology and comprises genomic DNA; (ii) one or more proteins or peptides are bound onto the one or more cytosines of the provided genomic DNA dependent on the presence or absence of methylation of said one or more cytosines; (iii) one or more probes are hybridized onto one or more sequences of the provided genomic DNA; and (iv) at least one signal is detected, said signal being specific for the methylation or unmethylation of at least one cytosine in a specific sequence context, whereby a cytosine of a CpG position or two, at least two, three, four, five, seven, ten, fifteen, twenty, fifty, one hundred, one thousand or more cytosines of different CpG positions are detected methylation specifically and sequence specifically. Thereby the steps (i)-(iv) can be performed in any arbitrary order. In preferred embodiment, the steps are carried out in the order (i), (ii), (iii), (iv). In another preferred embodiment, the steps are carried out in the order (i), (iii), (ii), (iv).

In a preferred embodiment, said one or more CpG positions are part of a repetitive sequence element.

According to a preferred embodiment, said one or more cytosines of said one or more CpG positions are encoded in the sequence of a repeat of a repetitive sequence element. This embodiment has the advantage, that corresponding cytosines will also be detected simultaneously, said cytosines being encoded by other repeats of said repetitive sequence element. This leads to a great enhancement of the signal(s) of step (iv) or (d).

In a preferred embodiment, the providing of a sample comprises at least one of the following: section, histological section, tissue section, paraffin-embedment, fixation, formalin-fixation, one or more cells, cell culture, or biopsy.

According to a preferred embodiment, a sample is provided, said sample comprises genomic DNA and has a conserved morphology. Thereby this sample can be any kind of sample such as, but not limited to, any kind of cell or tissue sample. Said cell or tissue sample is obtained from a culture or from an individual via a biopsy. Preferably said provided sample is a section. Any kind of section is usable according to the invention. In particular, it is possible to use any kind of section as it is routinely generated for histological analysis (histological section). Therefore it might be suitable to fix the sample. A person skilled in the art knows a lot of suitable fixatives and corresponding protocols for fixation (Dabbs DJ. "Diagnostic Immunohistochemistry", 2nd edition, Churchill Livingstone, Jan 2006). This can be done before and/or after sectioning of the sample. Accordingly, it is also possible to use archived samples, for example but not limited to paraffin-embedded formalin-fixed samples. Where suitable, it is also possible according to the invention to fix the sample in between the individual processing steps.

However, in any case, according to the method of the invention, the morphology is not destroyed of the one or more cells and/or tissue of the sample. According to the invention, a sample is not subjected to DNA isolation. In addition, a sample comprising isolated DNA is not useable according to the invention.

In a preferred embodiment, said protein or peptide comprises at least one of the following: antibody; anti-5-methylcytosine antibody; protein capable of methylation specifically binding DNA; MeCP 1; MeCP2; MBD; MBD2; MBD3; MBD4; Kaiso; CXXC-3 domain of MBD1; or any domain thereof.

According to a preferred embodiment, the protein or peptide which binds to the provided genomic DNA is any protein, peptide, or derivative thereof able to bind methylation specifically genomic DNA. Preferably, said protein is MeCP 1; MeCP2; MBD; MBD2; MBD3; MBD4, Kaiso, the CXXC-3 domain of MBD1 or any domain or peptide thereof. Artifically designed proteins, peptides, Spiegelmere or ribozymes, specfic for the binding of methylated CpG dinucleotides are useable as well. Of course and also preferred are also methylation sensitive restriction enzymes or methyltransferases, preferably only the DNA binding domains of the said or only peptides thereof. According to a preferred embodiment, the protein or peptide which binds to the provided genomic DNA is any antibody which binds methylation specifically onto genomic DNA. Such antibodies are known to those skilled in the art, for example but not limited to, the anti-5-methylcytosine antibody produced by Abcam (Abcam Inc; One Kendall Square; Bldg. 200, 3rd Floor; Cambridge, MA02139).

In a preferred embodiment, said one or more proteins or peptides are labeled

According to a preferred embodiment, the methylation specifically binding at least one protein or peptide is labeled.

In a preferred embodiment, two or more proteins or peptides are labeled with the same label or with a different label.

According to a preferred embodiment, one protein or peptide or 2, 3, 4, 5, 6, 7, 8, 9, 10, 15, 20 or more different proteins or peptides bind methylation specifically to the provided genomic DNA. Preferably, the said proteins or peptides are labeled differently. According to a preferred embodiment subgroups of a protein or peptide are labeled differently. According to another preferred embodiment, the two or more proteins or peptides have the same label.

In a preferred embodiment, said probe comprises at least one of the following: oligonucleotide; oligoribonucleotide; PNA-oligomer; LNA-oligomer; or any derivate thereof.

According to a preferred embodiment, the probe which hybridizes onto the provided genomic DNA is any kind of probe which is able to bind sequence specifically onto a nucleic acid. Preferably each molecule of said probe is labeled by two or more labels which leads to an enhancement of the said signal. Preferably, said probe is an oligonucleotide, an oligoribonucleotide, a PNA oligomer, or any derivative thereof comprising also any chimeric molecule thereof.

Preferably, probes of any length are useable according to the invention as long as they sequence specifically bind on to DNA. According to a particular preferred embodiment, the probe length is between 100 nucleotides and 10.000 nucleotides. According to a particular preferred embodiment, the probe length is between 50 nucleotides to 150 nucleotides. Preferably said probes are generated by means of cloning and/or amplification such as but not limited to PCR. According to a preferred embodiment, said probes are oligonucleotides, an oligoribonucleotide, a PNA oligomer, or any derivative thereof. Preferably said probes are generated by artificial synthesis. According to a particular preferred embodiment, the probe length is between 8 nucleotides and 50 nucleotides. Preferable it is 8, 10, 15, 20, 25, 30, 35, 40, 45, or 50 nucleotides in length. Also preferably, said probes are generated by artificial synthesis.

In a preferred embodiment, said one or more probes are labeled.

According to a preferred embodiment, the sequence specifically binding at least one probe is labeled.

In a preferred embodiment, two or more probes are labeled with the same label or with a different label.

According to a preferred embodiment, one probe or 2, 3, 4, 5, 6, 7, 8, 9, 10, 15, 20 or more different probes bind sequence specifically to the provided genomic DNA. Preferably, the said probes are labeled differently. According to a preferred embodiment subgroups of a probe are labeled differently. According to another preferred embodiment, the two or more probes have the same label.

In a preferred embodiment, the protein or peptide label and/or the probe label are a fluorescent dye, a luminescent dye, an antigen, an antibody, a ligand, a ligand-binding molecule, a biotin molecule, a streptavidin molecule, an oligonucleotide, a radioactive label, a mass label, or a reagent.

According to a preferred embodiment, any kind of label known to those skilled in the art is useable for labeling the said one or more proteins or peptides, the said one or more probes, or both. Preferably, the said label or labels are one or more fluorescent dyes such as but not limited to Cy5, Cy3; one or more luminescent dyes; one or more antigens; one or more antibodies; one or more ligands; one or more ligand binding molecules; one or more biotin molecules; one or more streptavidin molecules; one or more avidin molecules; one or more oligonucleotides comprising also oligoribonucleotides, PNA oligomers, derivates thereof, in particular chimeric molecules thereof; one or more fluoresent or luminescent proteins or peptides, one or more green fluorescent protein or a derivative thereof; one or more radioactive labels; one or more mass label; one or more chemical reagent; one or more particle label comprising amongst other metal particle labels such as but not limited to gold, or silver particle labels; or combinations of the said. According to a preferred embodiment, said labels are used in combination with suitable signal enhancing substances such as scintillators for radioactive labels. A person skilled in the art is able to the select labels from the said possible labels or combinations thereof for achieving the desired sensitivity.

In a preferred embodiment, a fluorescence resonance energy transfer (FRET) or a bioluminescence resonance energy transfer (BRET) takes place between the label of the protein or peptide and the label of the probe.

According to a preferred embodiment, at least one protein or peptide is labeled with a fluorescent dye and at least one probe is labeled with a different fluorescent dye. A fluorescence resonance energy transfer (FRET) takes place between the said different fluorescent labels, wherein the said labels are in proximity of each other. According to a preferred embodiment, at least one protein or peptide is labeled with a luminescent dye and at least one probe is labeled with a different luminescent dye. A luminescence resonance energy transfer (BRET) takes place between the said different luminescent labels, wherein the said labels are in proximity of each other. The said labeling with said fluorescent or bioluminescent dyes might be a direct labeling of the protein or peptide or probe, or might be an indirect labeling of the protein or peptide or probe via any known linking molecule(s) such as but not limited to antigens, antibodies, ligands, ligand binding molecules, biotin molecules, streptavidin molecules, or avidin molecules.

In a preferred embodiment, (a) the protein or peptide and the probe are each labeled with a reagent, (b) the two reagents react with each other enabled by the binding of the protein or peptide and by the hybridizing of the probe, and (c) the product of said reaction is detected.

According to a preferred embodiment, a methylation specifically binding protein or peptide is labeled with a reagent and a sequence specifically binding probe is labeled also with a reagent. Preferably said reagents are the same, preferably said reagents are different reagents. In particular preferred embodiment, said reagents are molecules which have the ability to react with each other. According to a preferred embodiment, said reagents react with each other and the product of the reaction is detectable. According to a preferred embodiment, the product of said reaction is detected by any suitable means known those skilled in the art. Such means are for example but not limited to antibodies, fluorescence, luminescence, molecules which bind the product, proteins, peptides, or combination thereof.

In a particular preferred embodiment, one reagent is tryptophan and the other reagent is 2-hydroxy-5-nitrobenzyl bromide.

According to a particularly preferred embodiment, the said protein or peptide is labeled with tryptophan and the said probe is labeled with 2-hydroxy-5-nitrobenzyl bromide. The tryptophan and the 2-hydroxy-5-nitrobenzyl bromide are brought into proximity with each other by a binding of the said protein or peptide and the said probe in proximity to each other on the provided genomic DNA. This enables a chemical reaction which results in fluorescent dye. Said dye is detectable at 420 nm, a wave length at which tryptophan is not absorbing (Barman TE, Koshland DE Jr. A colorimetric procedure for the quantitative determination of tryptophan residues in proteins. J Biol Chem. 1967 Dec 25;242(23):5771-6.; Horton HR, Koshland DE Jr. A highly reactive colored reagent with selectivity for the tryptophan residue in proteins. 2-hydroxy-5- nitrobenzyl bromide. J Am Chem Soc. 1965 Mar 5;87:1126-32).

In a preferred embodiment, said at least one detectable signal is generated by means of applying at least one of the following with respect to the binding of the one or more proteins or peptides and/or with respect to the hybridization of the one or more probes: secondary antibody, antibody binding protein, complement factor, ligand, ligand-binding protein, biotin, streptavidin, avidin, antigen, antigen-binding antibody, fluorescent or luminescent protein or peptide, green fluorescent protein or derivative thereof, protein tag, His-tag, digoxigenin, FLAG-tag, hemagglutinin tag, glutathion, glutathion S-transferase, S-tag, S protein, tag-binding protein, padlock probe, ligase, polymerase, peroxidase, alkaline phosphatase, fluorescent dye, luminescent dye, radioactive label, or mass label.

According to a preferred embodiment, one or more signals are obtained depending on the number of differently labeled probes. Each of said signal is derived by the proximate binding of the said protein or peptide and the said probe. According to a preferred embodiment an amplified signal is derived. Therefore one or more of the following is used: secondary antibody, antibody binding protein, complement factor, ligand, ligand-binding protein, biotin, streptavidin, avidin, antigen, antigen-binding antibody, protein tag, His-tag, digoxigenin, FLAG-tag, hemagglutinin tag, glutathion, glutathion S-transferase, S-tag, S protein, fluoesent or luminescent protein or peptide, green fluorescent protein or a derivative thereof, tag-binding protein, padlock probe, ligase, polymerase, peroxidase, alkaline phosphatase, fluorescent dye, luminescent dye, radioactive label, or mass label. Thereby the said may be applied to the said protein or peptide, to the said probe, or both.

In a preferred embodiment, (a) firstly, at least one protein or peptide is bound to the provided genomic DNA, wherein said binding is dependent on the methylation status of the binding site; (b) secondly, at least one probe is hybridized onto the provided genomic DNA, wherein said hybridizing is dependent on the sequence of the hybridization site and on the absence of said at least one protein or peptide from the hybridization site; (c) thirdly, at least one genomic or artificial region is amplified by means of at least one of the hybridized probes; (d) fourthly, at least one signal is detected, said signal being specific for the amplificates, wherein the amplificates represent the methylation status of at least one or more CpG positions of a specific sequence.

According to a preferred embodiment, a genomic or artificial region is amplified. Thereby (i) one or more proteins or peptides bind methylation specifically onto the provided DNA; (ii) one or more probes bind sequence specifically onto said DNA; (iii) one or more genomic or artificial regions are amplified, wherein said one or more probes are either used as primer or as template; (iv) the occurrence of respective amplifications is detected by detecting one or more corresponding signals which are specific for the amplificates of the different reactions. Because the amplificates are methylation specific and sequence specific, they allow a methylation specific detection of one or more CpG positions of specific one or more sequences. According to a preferred embodiment, the said steps are carried out in the order (i), (ii), (iii), (iv) or in the order (ii), (i), (iii), (iv).

In a particular preferred embodiment, (a) one or more of said probes are a padlock probe, or an extendable oligonucleotide or derivative thereof, and/or (b) said amplification comprises at least a ligase, a polymerase, or both.

According to particular preferred embodiment, the one or more sequence specific binding probes are padlock probes. Padlock probes are known to those skilled in the art. In brief, a padlock probe is a probe, comprising a site specific 5'-terminal region and a site specific 3'-terminal region. After hybridization of the probe to a site to which the probe is specific for, the 3'-terminal region is ligated or extended and ligated with the 5'- terminal region. Therefore a ligase, a polymerase, or both are used. Thus a circular molecule is generated which is hybridized to the site the probe is specific for. The said used circular molecule is then used for amplification. Alternatively in another preferred embodiment, a circular molecule is applied directly for hybridization in step (ii). According to a particular preferred embodiment, the one or more sequence specific binding probes are normal 3'-extendable probes. Suitable probes are known to those skilled in the art, for example but not limited to oligonucleotides or derivates thereof comprising amongst other chimeric probes of nucleotides, ribonucleotides or PNA-monomers. Preferably, one or two of said probes are used to generate one amplificate specific for each site.

In a preferred embodiment, (a) one or more proteins or peptides are associated via a linker with sequence specific padlock probes; (b) said padlock probes hybridize sequence specifically to the provided genomic DNA; (c) generation of one or more circular molecules by means of a ligase or of a polymerase and a ligase, the said connecting the 5' end and 3' end of the one or more padlock probes; (d) amplification of the one or more circular molecules by means of primer and polymerase; (e) one or more probes comprising the same sequence specificity as the one or more padlock probes are hybridized onto the amplified DNA; and (f) the at least one signal is detected, said signal is specific for the bound proteins or peptides specific for respective methylation, and for the bound probes specific for the respective one or more genomic sequences.

According to a preferred embodiment, the following steps are carried out: (I) A protein or peptide is methylation specifically bound to provided genomic DNA, wherein a sequence specific padlock probe is attached to said genomic DNA methylation specifically binding protein or peptide. Said attachment is either directly via a linker or indirectly by means of interaction with other proteins, peptides, nucleic acids, or any suitable ligands thereof. (II) The said padlock probe is sequence specifically bound to the provided DNA. (III) A circular nucleic acid molecule is obtained by joining sequence specifically the 3'end of the padlock probe with its 5'end. Therefore a ligase or a polymerase and a ligase is used depending on the distance of between the ends. Wherein the two ends hybridize on nucleotides directly adjacent to each other, a ligase is only necessary for joining the ends. Wherein the ends hybridize on nucleotides separated by one, two, three, four, five or more nucleotides, a polymerase first extends the 3'end before a ligase will connect the now immediately adjacent ends. (IV) The generated circular nucleic acid molecule is then amplified. Preferably by means of an isothermal amplification (rolling circle amplification). Said amplification method has the advantage that the amplificate is localized to the site of amplification, which avoids diffuse or even false positive signals. But of course, and also preferred, other amplification methods are possible. A person skilled in the art is aware of suitable methods, for example, but not limited to, PCR and LCR. (V) At least one amplificate specific probe is hybridized to the amplificate. Preferably, said probe has the same specificity as the padlock probe i.e. it encodes at least in parts the sequence which cause the specificity of the padlock probe. Preferably, said probe comprises at least in parts a sequence encoded by the padlock probe which is not part of the sequence specific site of it. However said probe is directly or indirectly labeled with a suitable dyes as they are known to those skilled in the art. For example, but not limited to, said dye is a fluorescent dye; a luminescent dye; a ligand; a ligand binding protein in particular an enzyme; an antigen; an antibody; and/or a fluorescent or luminescent protein or peptide. (VI) A signal is detected being derived from said label of the amplificate hybridized probes. Said signal is specific for the methylation or non-methylation of a specific sequence. Thus the methylation or non-methylation of a specific CpG position within a specific genomic sequence is detected. According to a preferred embodiment, the steps are carried out in the order: (I), (II), (III), (IV), (V), (VI) or in the order (II), (I), (III), (IV), (V), (VI). According to a preferred embodiment, 1, 2, 3, 4, 5, 6, 7, 8, 9, 10 or more proteins or peptides are bound methylation specifically to provided genomic DNA. According to a preferred embodiment, each of said proteins or peptides being attached with one padlock probe or with multiple different padlock probes. According to a preferred embodiment, each individual protein or peptide molecule carries one padlock probe molecule or multiple copies of it.

In a preferred embodiment, (a) one or more methylation specific proteins or peptides are associated via linker with one or more sequence specific probes; (b) at least one of said protein or peptide associated probes hybridize sequence specifically to the provided genomic DNA; (c) amplification by means of at least one of the said hybridized one or more probes; and (d) at least one signal is detected, wherein each of said signals is specific for the bound proteins or peptides which are associated with amplificates, and wherein each of said signals is specific for respective methylation and for respective genomic one or more sequences.

According to a preferred embodiment, the following steps are carried out: (I) A protein or peptide is methylation specifically bound to provided genomic DNA, wherein a sequence specific probe is attached to said genomic DNA methylation specifically binding protein or peptide. Said attachment is either directly via a linker or indirectly by means of interaction with other proteins, peptides, nucleic acids, or any suitable ligands thereof. (II) The said probe is sequence specifically bound to the provided DNA. (III) The said probe is used for amplification. This can be any kind of amplification for example but not limited to isothermal amplification, PCR or LCR. The resulting amplificates are either labeled directly or indirectly. A person skilled in the art knows suitable labels. For example, but not limited to, said dye is a fluorescent dye; a luminescent dye; a ligand; a ligand binding protein in particular an enzyme; an antigen; an antibody; and/or a probe such as a oligonucleotide, oligoribonucleotide, PNA oligomer or derivative thereof. (IV) A signal is detected being derived from said label specific for the amplificates. Said signal is specific for the methylation or non-methylation of a specific sequence. Thus the methylation or non-methylation of a specific CpG position within a specific genomic sequence is detected. According to a preferred embodiment, the steps are carried out in the order: (I), (II), (III), (IV) or in the order (II), (I), (III), (IV). According to a preferred embodiment, 1, 2, 3, 4, 5, 6, 7, 8, 9, 10 or more proteins or peptides are bound methylation specifically to provided genomic DNA. According to a preferred embodiment, each of said proteins or peptides being attached with one probe or with multiple different probes. According to a preferred embodiment, each individual protein or peptide molecule carries one probe molecule or multiple copies of it. It is advantageous to carry out the particular preferred embodiment of isothermal amplification by means of a probe associated with said protein or peptide, because the resulting signal is amplification localized and diffuse or even false positive signals are avoided.

In a preferred embodiment, said at least one signal is detected by means of a microscope, or a confocal microscope.

According to a preferred embodiment, a signal is determined by the methylation specifically bound protein or peptide and the sequence specifically bound probe. Said signal is detected by any suitable means as it is known those skilled in the art. The suitability of said means is thereby determined by the used labels. Preferably said means is a microscope, more preferably a fluorescence microscope, and in particular a confocal microscope. But also other means are possible and also preferred. For example, but not limited to, an electron microscope, wherein the used label(s) are gold or silver particles. If the case may be, said particles are of different sizes.

A preferred embodiment comprises, (a) providing a sample, said sample has a conserved morphology and comprises genomic DNA; (b) binding at least one protein or peptide onto the provided genomic DNA, wherein said binding is dependent on the methylation status of the binding site; (c) degrading of accessible DNA; (d) releasing the bound at least one protein or peptide from the not degraded DNA; (e) hybridizing one or more probes onto the provided genomic DNA, wherein said hybridizing is dependent on the sequence of the hybridization site; (f) detecting at least one signal derived from the genomic DNA hybridized onto the said one or more probes, wherein said one or more CpG positions are detected in a methylation specific and sequence specific manner. In a preferred embodiment, the signal intensity derived from the probe is used to normalize the signal intensity from the interactionof methylation specific protein and sequence specific probe. In that way a quantitative measurement of the DNA methylation is achieved.

According to a preferred embodiment, the following steps are carried out: (I) a sample is provided comprising genomic DNA. Said sample has also a conserved morphology. Preferably this step comprises the accessible making of DNA for the binding of proteins or peptides and/or for degradation. (II) One or more proteins or peptides are bound methylation specifically onto said DNA. Thereby DNA is obtained which is either covered by the bound protein(s) or peptide(s) or is uncovered. (III) Said uncovered DNA is digested by means as they are known to those skilled in the art. For example but not limited to, the uncovered DNA is digested by means of nucleases, endonucleases, restriction enzymes, exonucleases, or DNase. (IV) The said protein(s) or peptide(s) bound to the covered DNA are separated from said DNA. A person skilled in the art knows possibilities how to remove proteins or peptides from DNA. For example, but not limited to it, the said covered DNA comprising the bound protein(s) or peptide(s) is subjected to a protein digestion, a DNA purifying device, or chemical solvent. (V) At least one probe is sequence specifically bound onto the purified DNA of step (IV). (VI) The binding of said at least one probe onto the said purified DNA is detected. A person skilled in the art knows suitable means for detection. For example but not limited to amplification, isothermal amplification, PCR, LCR, NASBA, real time PCR, FACS, fluorescent dye, luminescent dye, radioactive dye, antigen, antibody, ligand, ligand-binding molecule, enzyme, microscope, fluorescence microscope, confocal microscope.

In a preferred embodiment, the amplification of the above described steps is performed by means of ligation, PCR, and/or isothermal amplification.

According to a preferred embodiment, the above described amplifications are carried out by any method or means as they are known to those skilled in the art. Preferably, amplification is carried out by means of PCR. A person skilled in the art knows suitable PCR methods. Preferably, the amplification is carried out by means of an isothermal amplification. Suitable isothermal amplification methods are known to those skilled in the art, for example but not limited to it, the primer extension. Preferably, the amplification is carried out by means of NASBA. NASBA methods are RNA-DNA based amplifications which comprise the use of a reverse transcriptase, a RNA polymerase and a RNase. A person skilled in the art knows suitable NASBA methods. Preferably, the amplification is carried out by means of a ligase chain reaction (LCR). In general, LCR methods are methods which are based on the use of a ligase. A person skilled in the art knows suitable LCR method.

In a preferred embodiment, the amplification is performed by means of real time PCR.

According to a preferred embodiment, a real time based amplification method is used for amplification. Preferably it is also used for detection. A person skilled in the art knows suitable real time based amplification and/or detection methods, for example but not limited to, the Taqman™ method, the Lightcycler™ method, the Scorpion™ method, the Headloop™ method, or the Molecular Beacon™ method.

In particular, aspects of the herein described embodiments are at least embodiments for one of the following: histochemistry, immunohistochemistry, pathology, histology, cell culture staining, cell biology analysis, optical imaging.

According to a preferred embodiment, the herein described embodiments are used in any field known to those skilled in the art. For example, but not limited to the herein described embodiments are used for histochemistry, immunohistochemistry, pathology, histology, cell culture staining, cell biology analysis, optical imaging.

Particular aspects of the invention provide a method for detecting the co-localization of two molecules, comprising (a) labeling one molecule with an educt of a reaction; (b) labeling of the other molecule with another educt of said reaction; (c) allowing a reaction in which the two educts take part, wherein the corresponding two molecules co-localized with each other so that the two educts become in close proximity with each other; and (d) and detecting the product of said reaction.

According to particular aspects of the invention, the method of the invention is a method detecting two molecules in close proximity. Said method comprises that therefore an educt of a reaction is attached to each of the said two molecules. Subsequently conditions are applied which enable said reaction of the educts, wherein the said two molecules are in close proximity to each other and therefore also the two educts. The product of said reaction is then detected, said product being indicative for a close proximity of the said two molecules. Of course, also the co-localization of more then two molecules can be detected by using three or more educts.

In a preferred embodiment, one educt is tryptophan and the other educt is 2-hydroxy-5-nitrobenzyl bromide. The tryptophan and the 2-hydroxy-5-nitrobenzyl bromide are brought into proximity with each other by a bringing the said two molecules into proximity with each other. This enables a chemical reaction which results in fluorescent dye. Said dye is detectable at 420 nm, a wave length at which tryptophan is not absorbing (Barman TE, Koshland DE Jr. A colorimetric procedure for the quantitative determination of tryptophan residues in proteins. J Biol Chem. 1967 Dec 25;242(23):5771-6.; Horton HR, Koshland DE Jr. A highly reactive colored reagent with selectivity for the tryptophan residue in proteins . 2-hydroxy-5- nitrobenzyl bromide. J Am Chem Soc. 1965 Mar 5;87:1126-32).

### Kit of the invention

Aspects of the present invention relate to a kit comprising (a) a container; (b) one or more proteins or peptides each of which specifically binding to a CpG position dependent on the methylation status of said position; and (c) one or more probes each of which specific for a genomic sequence.
Aspects of the present invention relate to a kit suitable for carrying out the method of the invention. Said kit comprises at least one container, at least one protein or peptide as specified herein, and at least one probe as specified herein. Each of said proteins or peptides is able to methylation specifically bind genomic DNA. Each of said probes is able to bind to DNA of a specific sequence.

Aspects of the present invention relate to a kit for carrying out the method of the invention comprising (a) a container; (b) one or more proteins or peptides each of which specifically binding to a CpG position dependent on the methylation status of said position; and (c) one or more probes each of which specific for a genomic sequence.

A preferred kit, comprises a description or manual for carrying out the method of the invention. A preferred kit is also a kit, which comprises a description or manual for carrying out one, two, three, four, five or more embodiments of the invention.

A preferred kit is a kit, wherein the said one or more proteins or peptides are at least one selected from the group comprising antibody, anti-5-methylcytosine antibody, protein capable of methylation specifically binding DNA, MeCP 1, MeCP2, MBD, MBD2, MBD3, MBD4, Kaiso, CXXC-3 domain of MBD1, or any domain thereof.

A preferred kit is a kit, wherein the said one or more probes are at least one selected from the group comprising oligonucleotide; oligoribonucleotide; PNA-oligomer; LNA-oligomer; padlock probe; or any derivate thereof.

A preferred kit is a kit, wherein the said one or more proteins or peptides are at least one selected from the group comprising antibody, anti-5-methylcytosine antibody, protein capable of methylation specifically binding DNA, MeCP 1, MeCP2, MBD, MBD2, MBD3, MBD4, Kaiso, CXXC-3 domain of MBD1, or any domain thereof; and/or wherein the said one or more probes are at least one selected from the group comprising oligonucleotide; oligoribonucleotide; PNA-oligomer; LNA-oligomer; padlock probe; or any derivate thereof.

A preferred kit is a kit, wherein the said one or more proteins or peptides are labeled.

A preferred kit is a kit, wherein the said one or more proteins or peptides are labeled with at least one of the following: fluorescent dye, luminescent dye, antigen, antibody, ligand, ligand-binding molecule, biotin molecule, streptavidin molecule, oligonucleotide, radioactive label, or mass label
A preferred kit is a kit, wherein the said one or more probes are labeled.

A preferred kit is a kit, wherein the said one or more probes are labeled with at least one of the following: fluorescent dye, luminescent dye, antigen, antibody, ligand, ligand-binding molecule, biotin molecule, streptavidin molecule, oligonucleotide, radioactive label, or mass label.

A preferred kit is a kit, wherein the said one or more proteins or peptides and the said one or more probes are labeled with at least one of the following: fluorescent dye, luminescent dye, antigen, antibody, ligand, ligand-binding molecule, biotin molecule, streptavidin molecule, oligonucleotide, radioactive label, or mass label.

A preferred kit is a kit, wherein the label of at least one of the said proteins or peptides and the label of at least one of the said probes enable a FRET or a BRET event.

A preferred kit is a kit further comprising at least one of the following: ligase; polymerase; primer; secondary antibody; antibody binding protein; complement factor; ligand; ligand-binding protein; biotin; streptavidin; avidin; antigen; antigen-binding antibody; protein tag; His-tag; digoxigenin; FLAG-tag; hemagglutinin tag; glutathion; glutathion S-transferase; S-tag; S protein; tag-binding protein; padlock probe; ligase; polymerase; fluorescent dye; luminescent dye; radioactive label; or mass label.

A preferred kit is a kit, wherein one or more proteins or peptides are labeled with a padlock probe.

### Use of a method or a kit of the invention

Particular aspects of the invention relate to the use of the method of the invention or to the use of a kit of the invention. In particular, the use of a method or kit of the invention is preferred for at least one of the following with regard to a patient or individual: diagnosing a condition, prognosing a condition, predicting a treatment response, diagnosing a predisposition for a condition, diagnosing a progression of a condition, grading a condition, staging a condition, classification of a condition, characterization of a condition, or combinations thereof, wherein the condition is a healthy condition or an adverse event, the adverse event comprises at least one category selected from the group comprising: undesired drug interactions; cancer diseases, proliferative diseases or therewith associated diseases; CNS malfunctions; damage or disease; symptoms of aggression or behavioral disturbances; clinical; psychological and social consequences of brain damages; psychotic disturbances and personality disorders; dementia and/or associated syndromes; cardiovascular disease of the gastrointestinal tract; malfunction, damage or disease of the respiratory system; lesion, inflammation, infection, immunity and/or convalescence; malfunction, damage or disease of the body as an abnormality in the development process; malfunction, damage or disease of the skin, of the muscles, of the connective tissue or of the bones; endocrine and metabolic malfunction, damage or disease; and headaches or sexual malfunction. Accordingly a nucleic acid is obtained from a sample derived from an individual or patient. Said nucleic acid is then analyzed according to the method of the invention. Preferably, therefore a kit of the invention is used. The results obtained by this analysis enable then the said diagnosing a condition, prognosing a condition, predicting a treatment response, diagnosing a predisposition for a condition, diagnosing a progression of a condition, grading a condition, staging a condition, classification of a condition, characterization of a condition, or combinations thereof.

In particular, the use of a method or kit of the invention is preferred for distinguishing cell types or tissue, or for investigating cell differentiation. Accordingly a nucleic acid is obtained ex vivo from samples of cells, cell types or tissue. Said nucleic acid is then analyzed according to the method of the invention. Preferably, therefore a kit of the invention is used. The results obtained by this analysis characterized the cells, cell types or tissue. Therewith the results enable the distinguishing of cell types or tissue, or the investigating of cell differentiation.

All herein cited documents are incorporated by reference to their entirety.

### BRIEF DESCRIPTION OF THE FIGURES

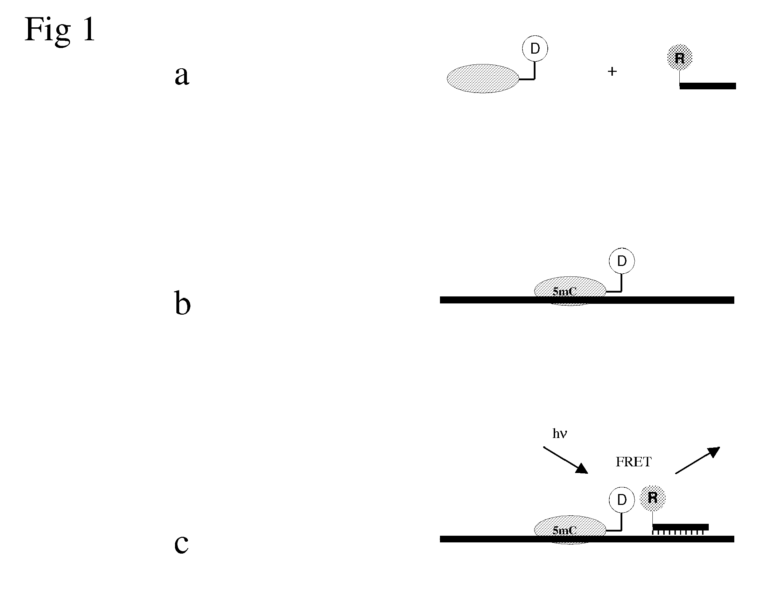

Figure 1 shows an overview of one embodiment, wherein a protein able to bind methylation specifically DNA and a probe able to hybridize sequence specifically DNA are provided (Fig 1 a). Said protein as well as said probe are labeled with a fluorescent dye, said pair of labels is suitable for a FRET reaction. Either first the protein (Fig 1 b) or the probe (not shown) is bound to genomic DNA of a sample (e.g. a tissue section derived from a formalin-fixed paraffin-embedded sample). Thereafter, the probe or respectively the protein is bound to said genomic DNA. In an alternative embodiment, said protein and probe are hybridized simultaneously onto the genomic DNA. Fig 1 c shows the principle of detection. Excitation light (h·v) of a certain wave length is applied to the sample, wherein the excitation light (h·v) is absorbed by the donor fluorescent dye, which transfers energy to the receptor fluorescent dyes, which on his part emits light of another wave length. Said signal is detected and is indicative for the presence of a methylated or unmethylated CpG position in the context of a specific sequence. In addition, Figure 1 illustrates the basic principle of the invention: A sequence specifically binding probe and a methylation specific binding protein or peptide bind in proximity to each onto genomic DNA. Thereby the protein or peptide and the probe are labeled with two different dyes. The protein or peptide as well as the probe is labeled directly or indirectly with one or more molecules of the respective dye. Finally, a signal is derived from the interference or co-localization of said dyes. This signal is indicative for the presence or absence of methylation in a specfic sequence context.

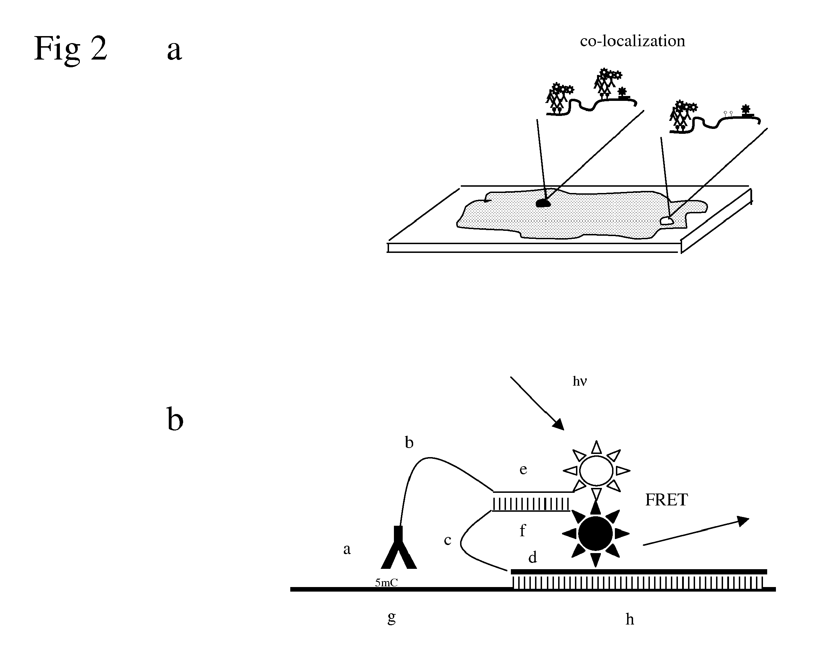

Figure 2 a shows an overview of one embodiment, wherein an antibody detects methylated cytosine (closed circle) and said bound antibody is detected on his part by a plurality of labeled secondary antibodies. Said label (open star) is for example Cy3. The probe sequence specifically bound is labeled with a different dye (closed star), for example Cy5. Thereby each probe or secondary antibody molecule may each carry a plurality of labeling molecules (fluorescent dye molecules). Wherein a cell comprises a methylated cytosine in the context of a specific sequence, a yellow signal is detectable in the nucleus of said cell. Wherein a cell comprises a unmethylated cytosine in the context of a specific sequence, no such a yellow signal is detectable, because the said primary antibody was not able to bind to respective unmethylated cytosine (open circle). A yellow signal is detected because of an co-localization of the co-localizing primary and secondary antibodies with the probe i.e. of the orange signal derived from Cy3 and red signal derived from Cy5.
Figure 2 b shows an overview of one embodiment, wherein an antibody (a) detects methylated cytosine (5mC, (g)) and a probe (d) is hybridized onto a adjacent specific sequence (h). The antibody as well as the probe are linked via respective linkers ((b), (c)) to each other complementary sequences ((e), (f)), wherein each sequence is labeled with a fluorescent dye able to perform a FRET reaction (open and closed stars; for example but not limited to, the open star represents fluorescein and the closed star represents tetramethylrhodamine). Only wherein said antibody binds adjacent to the sequence the probe is hybridized to, a signal is detectable by FRET. Wherein only the probe is able to bind or wherein only the antibody is able to bind, no signal is detectable.

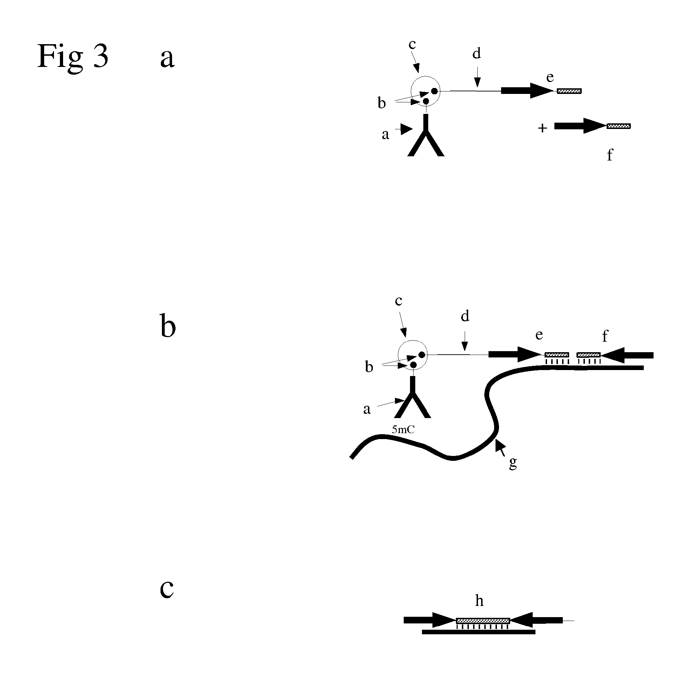

Figure 3 shows an overview of one embodiment. Fig 3a shows an antibody (a) able to bind to DNA methylation specifically is labeled with biotin (b). Said antibody is able to be associated with a probe (e) comprising a primer binding site (dark arrow) and a sequence specific region (gray bar). Said probe is labeled via a linker (d) also with biotin (b). The association of the probe and the antibody is achieved by addition of streptavidin or avidin (c) which binds the biotin labels. In addition, a second probe (f) is applied, said probe comprising a primer binding site (dark arrow) and a sequence specific region (gray bar). Fig 3b gives an overview of the embodiment after binding of the antibody and the probes onto the genomic DNA of a cell of a provided sample and after the association via the biotin labels. After associated of the two probes the gap between the two probes is closed by means of a ligase or if the case may be by means of a polymerase and a ligase. The product (h) of this is shown in Fig 3c. The connected probes are then detectable via amplification, e.g. PCR or LCR.

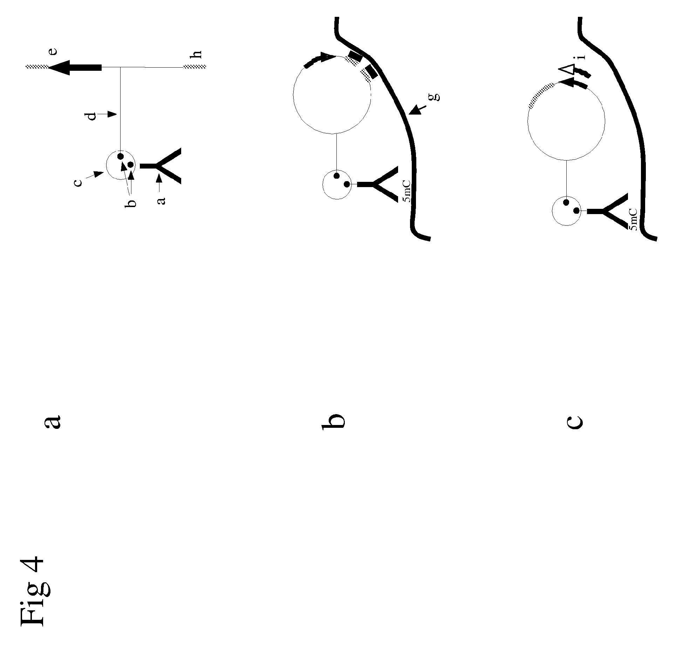

Figure 4 shows an overview of one embodiment. Fig 4a shows an antibody (a) able to bind to DNA methylation specifically is labeled with biotin (b). Said antibody is able to be associated with a padlock probe comprising at least one primer binding site (dark arrow) and two sequence specific regions (gray bars) at the 5' or 3' terminal region of the probe. Said probe is labeled via a linker (d) also with biotin (b). The association of the probe and the antibody is achieved by addition of streptavidin or avidin (c) which binds the biotin labels. Fig 4b gives an overview of the embodiment after binding of the antibody and the padlock probe onto the genomic DNA of a cell of a provided sample and after the association via the biotin labels. After associated of the padlock probe, the gap between the two ends are closed by means of a ligase or if the case may be by means of a polymerase and a ligase creating a circular molecule (Fig 4c). Said circular molecule is then amplified by means of a primer (i), e.g. in a rolling circle amplification. In a particular preferred embodiment, methylation specificity is enhanced by stringent washing steps, wherein probe/antibody complexes are removed that bind only sequence specifically onto the DNA. During such washing steps, probe/antibody complexes remain bound to the DNA, wherein both the probe and the antibody bind specifically to the provided DNA.

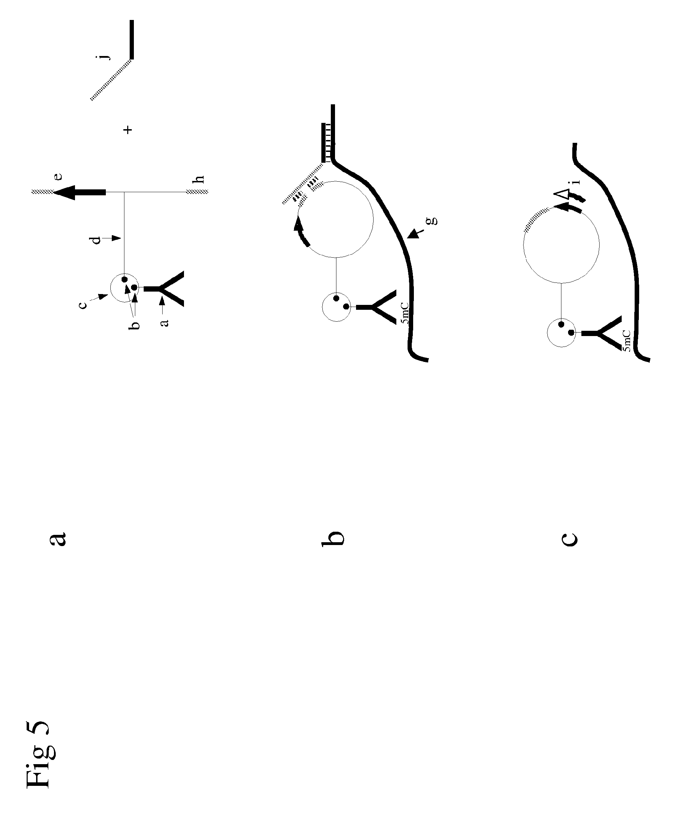

Figure 5 shows an overview of one embodiment. Fig 5a shows an antibody (a) able to bind to DNA methylation specifically is labeled with biotin (b). Said antibody is able to be associated with a padlock probe comprising at least one primer binding site (dark arrow) and two sequence specific regions (gray bars) at the 5' or 3' terminal region of the probe. Said probe is labeled via a linker (d) also with biotin (b). The association of the probe and the antibody is achieved by addition of streptavidin or avidin (c) which binds the biotin labels. In addition a second probe (j) is applied comprising a sequence specific region (black bar) and a padlock specific region (gray bar). Fig 5b gives an overview of the embodiment after binding of the antibody. the padlock probe, and the second probe onto the genomic DNA of a cell of a provided sample and after the association via the biotin labels. After hybridization of the padlock probe to the padlock probe specific region of probe (j), the gap between the two ends of the padlock probe are closed by means of a ligase or if the case may be by means of a polymerase and a ligase creating a circular molecule (Fig 5c). Said circular molecule is then amplified by means of a primer (i), e.g. in a rolling circle amplification.

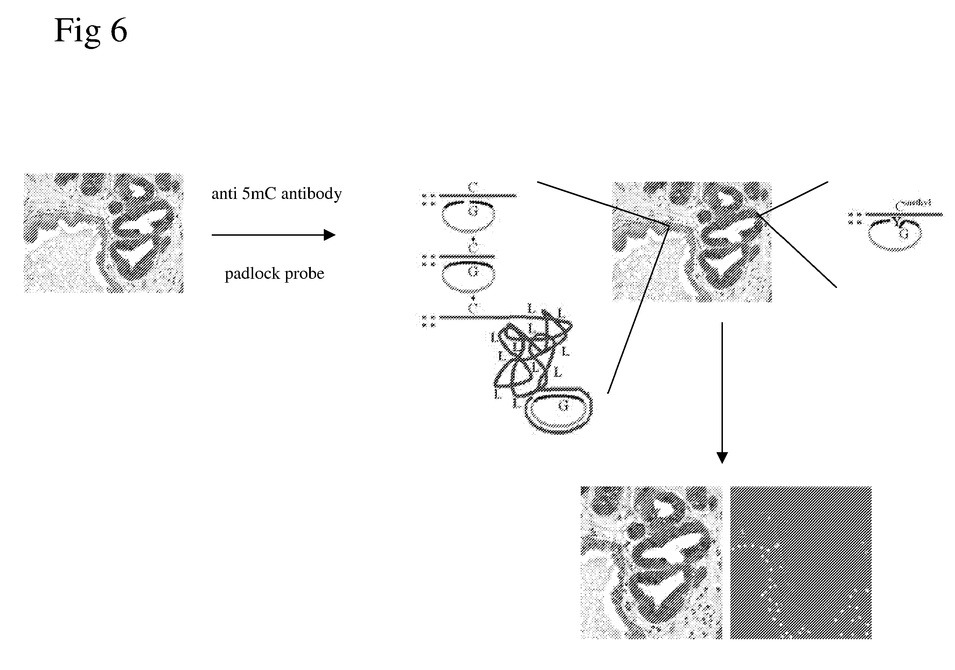

Figure 6 shows an overview of one embodiment. In an optional, first or last step, an immunohistological staining and analysis is performed. This facilitates the identification of target cells. If the case may be, images of regions of interest are taken. If desired, the sample is destained. In the second step, an antibody specific for methylated cytosine is applied followed by the application of a sequence specific padlock probe. Said padlock probe is not able to bind completely to a specific sequence, wherein an antibody is bound to the CpG that is part of the complementary sequence for which the two ends of the padlock are specific for. Therefore the two ends of the padlock probe can not be connected (middle picture left schematic drawing). On the other hand, the padlock probe is able to bind to a specific sequence, wherein no antibody is bound to the CpG position that is part of the complementary sequence for which the two ends of the padlock are specific for (middle picture, right schematic drawing). Thereafter the two ends of the padlock probe are connected with each other by means of a ligase or by means of a polymerase and a ligase forming a circular molecule. Said circular molecule is then amplified, e.g. by means of a rolling circle amplification for example but not limited to as described by Larsson C., et al, Nat Methods 2004 Dec1 (3):227-32. The detection occurs via labeled probes (L) specifically binding to the amplificate. The signal derived from said probes is then detectable as shown in the lower two pictures. The right pictures shows a staining obtainable by fluorescent labels. This can be compared with the picture obtained after immunohistological staining, e.g. by overlaying the two pictures and identifying cells having a unmethylated CpG within a specific sequence. Alternative the label of the probes is an enzyme suitable for staining. Said enzyme is for example but not limited to alkaline phosphatase. After the respective colorimetric reaction, a picture is obtainable as can be seen on the lower left. Cells having a unmethylated CpG within a specific sequence are stained (dark spots) within the immunohistochemical staining.

### DEFINITIONS

In particular aspects, the term "methylation status" refers to, but is not limited to, the presence or absence of methylation of a single nucleotide in a single DNA molecule, said nucleotide being capable of being methylated.

In particular aspects, the terms "luminescent" or "bioluminescent" refer to any kind of substance emitting light, said light being produced by means other than heat incandescence, e.g. by phosphorescence, fluorescence, or bioluminescence. The terms "luminescent" and "bioluminescent" are used herein as synonym.

### EXAMPLES

### Example 1: Usage of combined hybridization of 5mC-specific antibody and a sequence specific probe to detect DNA-methylation at histopathology sample slides

Slides on which paraffin-embedded tissue was mounted are incubated in 2 x SSC for one hour at 37°C. Afterwards they are dehydrated through a graded ethanol series at room temperature (RT), and air dried. Slides are incubated in 0.4% pepsin, freshly dissolved in warm (37°C) 0.9% NaCl (ph 1.5) for 15 min, dipped in Dulbecco's phosphate buffered saline (DBPS) at RT for 12 second, incubated in 1% hydroxylamine HCL at RT for 17 min, dipped again in DBPD, and incubated with 200 µl 4% paraformaldehyde under a plastic coverslip at room temperature for 10 min. This is followed by three washing steps with 2x SSC for 2 min at RT, and dehydration through a ethanol series. Denaturing is conducted in 70% formamide in 1x SSPE (ph 7) at 70°C for 3 min., and slides are plunged immediately into cold (-20°C) 70% ethanol. Slides are dehydrated through a cold graded ethanol series, air-dried, and placed on a 50°C slide warmer. Probe A is dissolved at 0.5 µM in 1x SSPE. 50 µl probe mixture is applied to prepared slides and immediately covered with a 22x22 mm coverslip (Eppendorf). Slides are allowed to hybridise for 18 h (over night). Coverslip is removed and slides are washed with gently agitation in 1x SSPE and 0.5xSSPE for 2 min at 50°C and dipped in water. Slides are air-dried and the DNA is baked to the slide by incubating at 95°C for 1 min. The slides are subsequently washed with PBS and incubated in blocking solution (2% BSA in PBT) for 2h at 37°C. Slides are incubated in a previous prepared dilution of antibody against 5-methyl cytosine conjugate in blocking solution for 1h at 37° and washed in blocking solution 3x at 37°c for 5 min each.
Antibody conjugate is prepared by incubating a 1 to 100 dilution of 5-methyl cytosine primary antibody (Eurogentec) with streptavidine and oligonucleotide B in PBS for 1h at 37°C. Subsequent conjugates are purified by Y50 Microcon columns and used for analysis.
Slip cover is removed and slides are washed with water and a RCA mixture is added: 50 mmol/I Tris-HCl (pH 7.5), 10 mmol/l MgCl₂, 20 mmol/l (NH₄)₂SO₄, 1 mmol/I dithiothreitol and 0.2 µg/µl BSA, 0.25 mmol/I dNTP, 1 to 10 000 diluted SYBRGreen, 2 ng/µl Phi 29 DNA polymerase and 0.1 pmol primer P1. The reactions is heated to 65°C and cooled to room temperature prior to addition of polymerase, followed by incubation at 37°C for 2h.
The slide is air-dried and analysed in a fluorescence microscope.

Proba A: 5'-GTGTTGACTTTGAGTTGTTTtcctgctgtctgtttactcc-3'
(in capitel letters: padlock specific sequence; in lower case: sequence specific for Homo sapiens glutathione S-transferase pi (GSTP1) gene (AY324387))

Oligonucleotide B (Padlock probe): 5'-Phosphate-aagtcaacacTTTTTTTTTTATGTTAAGTGACCGGCAGCATTTTTTTTTTaaacaactca-3'
(in capitel letters: probe specific sequence; in lower case: oligonucleotide P1 (primer) specific sequence

Oligonucleotide P1 (primer): 5'-TGCTGCCGGTCACTTAACAT-3'

Example 2: Usage of combined histological analysis and *in situ* genotyping (with 5-methylcytosine(5mC)-specific antibody and specific padlock probe) to relate methylation of specific DNA sequences to tissue architecture and specific cell types in tissue sections.

### A) Histological analysis

Several variants are possible:
1) Methylene Blue staining is performed according to standard histology protocols.
2) Immunohistochemical analysis.

Tissue sections from paraffin blocks are cut on slides dewaxed / rehydrated and after microwave pretreatment. Alternatively, fresh frozen tissue sections can be used. The sections are immunostained using primary antibodies such as anti-PSA, applied for 45 min at room temperature) with appropriate positive and negative controls. Appropriate biotinylated secondary antibody and streptavidin-peroxidase complexes are added. Detection is done using 3-amino-9-ethylcarbazole (Vector Laboratories, Burlingame, CA) as the chromogen.
1) and 2) can be done alternatively in adjacent tissue sections or applied to the same section.

Digital imaging is done also recording image coordinates.
The staining is removed using standard methods.

### B) In situ genotyping:

The destained tissues are treated with proteinase K (20 µg/ml), (Roche) for 2 hours at 37°C, washed with PBS, incubated for 2 h at 37 °C in a blocking solution (2% BSA in PBT), immuno labeled with anti 5-methyl cytosine antibody for 1 h at 37 °C, washed in blocking solution 3x at 37 °C for 5 min each and fixed for 10 min in 1% PFA to stabilize DNA-antibody complexes and washed with PBS. Then *in situ* genotyping is done according to Larsson C et al (Nat Methods 2004 Dec1 (3):227-32.)

The following reactions are carried out in a 50 µl reaction volume under a cover slip, or in reaction chambers defined by a silicone mask placed directly onto the slides.
Target sequences are made accessible for hybridization by digestion with a combination of restriction and exonuclease enzymes. The DNA is first restriction digested using 0.5 U/µl of restriction enzymes as Msd (New England Biolabs) or EcoRV (AP Biosciences) at 37 °C for 30 min in the corresponding supplied buffers supplemented with 0.2 µg/µl BSA (New England Biolabs). Then the slides are rinsed in buffer A (0.1 mol/I Tris-HCl pH 7.5, 0.15 mol/I NaCl and 0.05 % Tween-20). The ends of the restriction fragments are made single-stranded by exonucleolysis, using 0.2 U/µl of either the 3'-5' exonuclease III (New England Biolabs) or the 5'-3' A, exonuclease (New England Biolabs), at 37 °C for 15 min in the corresponding supplied buffers supplemented with 0.2 µg/µl BSA and 10 % glycerol. After incubation the slides are rinsed in buffer A.

Then 100 nmol/I of the padlock probe, designed to hybridize to the target sequence and also including sequences complementary to the target sequence of the detection oligonucleotide probes, are hybridized in a solution of 2 x SSC, 20 % formamide and 0.5 µg/µl sonicated salmon sperm DNA at 37 °C for 15 min.

Excess probe is removed by a wash in buffer B (2x SSC, 0.05 % Tween-20) for 5 min at 37 °C and then a rinse in buffer A. Padlock probes are circularized in 10 mmol/I Tris-acetate pH 7.5, 10 mmol/l MgAc₂, 250 mmol/I NaCl, 1 mmol/I ATP, 0.2 µg/µl BSA and 0.1 U/µl T4 DNA ligase (Amersham Biosciences) at 37 °C for 15 min. Slides for RCA detection are washed once in buffer B for 5 min at 37 °C, rinsed once in buffer A and dehydrated in a series of 70%, 85% and 100% ethanol.

The Rolling circle amplification (RCA) reactions are performed on dehydrated slides in 50 mmol/I Tris-HCl pH 7.5, 10 mmol/I MgCU, 20 mmol/l (NH₄)₂SO₄, 0.2 µg/µl BSA, 1 mmol/I DTT, 0.25 mmol/I dNTP, 10 % glycerol and 1 U/µl phage phi29 DNA polymerase (Fermentas) at 37 °C for 30 min in the silicone mask reaction chambers placed on a shaking platform. After polymerization, the masks are removed and the slides are rinsed in buffer A. The single-stranded RCA products are detected by hybridizing 250 nmol/I of Lin16 and Lin33 fluorescence-labeled oligonucleotide probes in a solution of 2x SSC, 20 % formamide and 0.5 µg/µl salmon sperm DNA for 15 min at 37 °C. After a brief rinse in buffer A, dehydrated the slides are finally mounted in Vectashield (Vector Laboratories) containing 10 ng/ml DAPI and stored at 4 °C. Alternatively to fluorescence-labeled oligonucleotide probes, biotin-labeled oligonucleotide probes can be used followed by the addition of streptavidin-peroxidase complexes and detection with non-fluorescent chromogen such as 3-amino-9-ethylcarbazole (Vector Laboratories, Burlingame, CA).

Digital imaging is done also recording image coordinates.

A) and B) image overlay is done for images with identical coordinates providing combined information on histology plus in situ genotyping.

## Claims

1. A method for detection of one or more CpG positions, comprising:
(a) providing a sample, said sample has a conserved morphology and comprises genomic DNA;
(b) binding at least one protein or peptide onto the provided genomic DNA, wherein said binding is dependent on the methylation status of the binding site;
(c) hybridizing at least one probe onto the provided genomic DNA, wherein said hybridizing is dependent on the sequence of the hybridization site;
(d) detecting at least one signal, each of said signals being determined by one or more of said bound proteins or peptides and by one or more of said hybridized probes, wherein said one or more CpG positions are detected in a methylation specific and sequence specific manner.

2. A method of claim 1, wherein said protein or peptide comprises at least one of the following: antibody; anti-5-methylcytosine antibody; protein capable of methylation specifically binding DNA; MeCP 1; MeCP2; MBD; MBD2; MBD3; MBD4; Kaiso; CXXC-3 domain of MBD1; or any domain thereof.

3. A method of claim 1, wherein said one or more proteins or peptides are labeled.

4. A method of claim 1, wherein said probe comprises at least one of the following: oligonucleotide; oligoribonucleotide; PNA-oligomer; LNA-oligomer; or any derivate thereof.

5. A method of claim 1, wherein said one or more probes are labeled.

6. A method of claim 3 and 5, wherein the protein or peptide label and/or the probe label are a fluorescent dye, a luminescent dye, an antigen, an antibody, a ligand, a ligand-binding molecule, a biotin molecule, a streptavidin molecule, an oligonucleotide, a radioactive label, a mass label, or a reagent.

7. A method of claim 6, wherein
(a) the protein or peptide and the probe are each labeled with a reagent,
(b) the two reagents react with each other enabled by the binding of the protein or peptide and by the hybridizing of the probe, and wherein
(c) the product of said reaction is detected.

8. A method of claim 7, wherein one reagent is tryptophan and the other reagent is 2-hydroxy-5-nitrobenzyl bromide.

9. A method of claim 1, comprising:
(a) providing a sample, said sample has a conserved morphology and comprises genomic DNA;
(b) binding at least one protein or peptide onto the provided genomic DNA, wherein said binding is dependent on the methylation status of the binding site;
(c) degrading of accessible DNA;
(d) releasing the bound at least one protein or peptide from the not degraded DNA;
(e) hybridizing one or more probes onto the provided genomic DNA, wherein said hybridizing is dependent on the sequence of the hybridization site;
(f) detecting at least one signal derived from the genomic DNA hybridized onto the said one or more probes, wherein said one or more CpG positions are detected in a methylation specific and sequence specific manner.

10. A method for detecting the co-localization of two molecules, comprising
(a) labeling one molecule with an educt of a reaction;
(b) labeling of the other molecule with another educt of said reaction;
(c) allowing a reaction in which the two educts take part, wherein the corresponding two molecules co-localized with each other so that the two educts become located in close proximity with each other; and
(d) and detecting the product of said reaction.

11. A kit, comprising
(a) a container;
(b) one or more proteins or peptides each of which specifically binding to a CpG position dependent on the methylation status of said position; and
(c) one or more probes each of which specific for a genomic sequence.
